Europäisches Patentamt

European Patent Office

Office européen des brevets

(19)

(11) **EP 1 233 748 B1**

(12) **FASCICULE DE BREVET EUROPEEN**

(45) Date de publication et mention
de la délivrance du brevet:
**26.01.2005 Bulletin 2005/04**

(21) Numéro de dépôt: **00985367.2**

(22) Date de dépôt: **30.11.2000**

(51) Int Cl.$^7$: **A61K 7/48**, A61P 17/00

(86) Numéro de dépôt international:
**PCT/FR2000/003345**

(87) Numéro de publication internationale:
**WO 2001/039739 (07.06.2001 Gazette 2001/23)**

(54) **ASSOCIATION RETINAL ET ALDEHYDES AVEC ACTIVITE ANTIFUNGIQUE**

ZUSAMMENSETZUNG MIT RETINAL UND ALDEHYDE MIT PILZVERHÜTENDER AKTIVITÄT

RETINALDEHYDE COMBINATION WITH ANTIFUNGAL ACTIVITY

(84) Etats contractants désignés:
**AT BE CH CY DE DK ES FI FR GB GR IE IT LI LU
MC NL PT SE TR**

(30) Priorité: **03.12.1999 FR 9915255**

(43) Date de publication de la demande:
**28.08.2002 Bulletin 2002/35**

(73) Titulaire: **Pierre Fabre Dermo-Cosmetique
92100 Boulogne (FR)**

(72) Inventeurs:
• **JEANJEAN, Michel
F-31320 Castanet-Tolosan (FR)**
• **LAGARDE, Isabelle
F-31540 Maurens (FR)**

• **SAURAT, Jean-Hilaire
CH-1206 Genève (CH)**

(74) Mandataire: **Ahner, Francis et al
Cabinet Régimbeau
20, rue de Chazelles
75847 Paris cedex 17 (FR)**

(56) Documents cités:
**EP-A- 0 391 033**          **WO-A-95/26709**
**WO-A-98/14167**          **WO-A-98/25587**
**US-A- 5 756 109**

• **HANDOJO I ET AL: "The effect of topical retinoic
acid (Airol) in the treatment of tinea versicolor."
SOUTHEAST ASIAN JOURNAL OF TROPICAL
MEDICINE AND PUBLIC HEALTH, (1977 MAR) 8
(1) 93-8. , XP000937595**

## Description

La présente invention concerne de nouvelles compositions utilisables en tant que médicament ou produit cosmétique comprenant une combinaison synergique d'un rétinoïde (de préférence le rétinaldéhyde) et d'aldéhydes linéaires, ces compositions présentant une activité antifongique sur Malassezia.

L'invention concerne également une composition topique destinée à être appliquée sur la peau ou le cuir chevelu, comprenant la combinaison active précitée dans un excipient acceptable en dermatologie ou cosmétologie.

Les champignons et levures parasites sont impliqués dans de nombreuses affections dermatologiques courantes, occasionnées par des désordres de la kératinisation notamment au niveau du cuir chevelu. Ces désordres entraînent l'apparition d'états pathologiques squamiformes comme c'est le cas, par exemple, dans les pellicules ou la dermite séborrhéique.

Ces états squameux sont très souvent associés à un processus inflammatoire localisé, entraînant l'apparition de prurit et de démangeaisons.

L'étiologie de ces états n'est pas totalement élucidée et elle est vraisemblablement sous la dépendance de différents facteurs.

Les connaissances actuelles indiquent que Malassezia, levure commune à tous les cuirs chevelus, est particulièrement impliquée dans ces pathologies et il est clairement établi aujourd'hui que la prolifération excessive de cette levure entraîne l'augmentation des états pelliculaires.

De plus, d'autres espèces de Malassezia viennent surenchérir l'état pelliculaire et supplanter la microflore cutanée physiologique, constituée essentiellement de bactéries Gram positif.

Il est également reconnu dans toute la littérature que le traitement des états squameux, directement liés à la prolifération de Malassezia, passe nécessairement par une démarche visant à diminuer le développement de la levure.

La prévalence du genre Malassezia chez les mammifères, étudiée de 1990 à 1993, a montré que le conduit auditif externe apparaissait comme la localisation préférentielle de ces levures. La présence de cérumen, riche en composites lipidiques, favorise la multiplication et la colonisation du germe (Guillot, 1994).

La peau contient à sa surface des composés lipidiques. Elle est aisément colonisée par ces mêmes levures lipophiles. Il est donc judicieux d'assainir les sites dont la colonisation excessive a conduit à un état pathologique accentué par une hyperséborrhée. L'emploi de molécules lipophiles, compatibles avec un véhicule favorisant la microbiodisponibilité des actifs choisis est une solution que nous proposons.

Dans l'état de la technique antérieur, certains dérivés undécyléniques ont déjà été proposés dans diverses applications antifongiques. De même, des molécules telles que les N Hydroxy pyridinones (ciclopiroxolamine, piroctone olamine), les sels de la pyrithione, le sulfure de sélénium, la classe des imidazolés, bien qu'efficaces sur diverses souches de Malassezia, ces molécules ne possèdent pas une action atrophique rapide sur l'épithélium cutané lorsqu'il est lésé par la colonisation d'un élément fongique.

Il est par ailleurs connu en microbiologie que le fait d'associer deux molécules différentes, actives sur le même germe, peut permettre d'aboutir à une activité résultante supérieure à celle obtenue avec chaque molécule prise séparément. Ceci peut s'expliquer par une diminution de la capacité de résistance du germe en présence des deux molécules associées et agissant toutes deux selon des mécanismes différents ou complémentaires.

De nombreuses molécules sont utilisées et formulées dans un but curatif mais pas leurs composantes rétinoïdiques, les formulations décrites dans la présente invention possèdent un réel intérêt pour une action préventive cutanée des états pelliculaires en particulier ceux décrits dans les dermites séborrhéiques.

Il existe donc un réel besoin pour un produit topique à propriétés antifongiques et qui posséderait également des propriétés trophiques pour les peaux lésées telles celles vues dans les dermatites séborrhéiques du visage, du cuir chevelu et des états pelliculaires en particulier des états stéatoïdes.

C'est pourquoi la présente invention a pour objet l'utilisation d'un aldéhyde linéaire couplé à un rétinoïde en particulier le rétinaldéhyde pour la préparation d'un médicament cosmétique topique ayant une activité fongistatique. Molécule intermédiaire dans le métabolisme des rétinoïdes naturels, le rétinaldéhyde exerce une activité sur les désordres cellulaires liés à des troubles de la différenciation épidermique.

Agissant comme régulateur de la différenciation épidermique, notamment par sa transformation enzymatique intra-kératinocytaire, le rétinaldéhyde peut présenter des propriétés intéressantes dans le traitement d'affections liées aux états érythémato-squameux tels que psoriasis, dermite séborrhéique du visage et du cuir chevelu ou états pelliculaires en général.

En plus de ses effets du type rétinoïde, le rétinaldéhyde possède une activité antimicrobienne que nous avons précédemment découverte. Cependant, il ne possède pas d'action notoire sur les levures telles Malassezia. Toutefois, exceptionnelles sont les molécules possédant une activité antimicrobienne et un autre type d'activité efficace sur l'épithélium cutané d'une part, et d'autre part, il y a un intérêt à associer des molécules à faible activité antimicrobienne, le gain d'activité antimicrobien étant plus probable.

Ainsi, la présente invention a pour objet une composition cosmétique contenant une combinaison d'au moins un rétinoïde et d'au moins un aldéhyde linéaire associée à au moins un excipient cosmétiquement acceptable, le rétinoïde étant de préférence le rétinaldéhyde, l'aldéhyde linéaire comprenant de préférence de 6 à 12 atomes de carbone bornes incluses, de manière encore plus préférée 11 atomes de carbone et plus particulièrement l'aldéhyde linéaire est l'aldéhyde undécylique, l'aldéhyde undécylénique ou un mélange de ces deux aldéhydes.

Selon une variante préférée, la composition cosmétique selon l'invention est telle que la concentration en rétinaldéhyde est comprise entre 0,001 et 0,5%, de préférence entre 0,015 et 0,25% en poids par rapport au poids total de la composition, et, la concentration en aldéhyde linéaire comprenant 11 atomes de carbone est comprise entre 0,001 et 0,5% et de préférence 0,03 et 0,5% en poids par rapport au poids total de la composition.

Ladite composition cosmétique peut en outre comprendre au moins un solvant choisi dans le groupe formé par les aldéhydes inférieurs (c'est-à-dire comprenant de 1 à 6 atomes de carbone) tels que le méthylal, les alcools inférieurs (c'est-à-dire comprenant de 1 à 6 atomes de carbone) tel que l'éthanol, les isoparaffines et plus particulièrement les chaînes comprenant de 6 à 12 atomes de carbone bornes incluses, les silicones linéaires et les silicones cycliques, et notamment le décaméthylcyclopentasiloxane, le diméthylpolysiloxane, l'hexaméthyldisiloxane.

L'invention concerne encore un procédé de préparation d'une composition cosmétique telle qu'on mélange au moins un rétinoïde, au moins un aldéhyde linéaire, au moins un excipient cosmétiquement acceptable et un procédé de traitement cosmétique comprenant l'application topique sur la peau ou le cuir chevelu d'une composition cosmétique, ledit traitement cosmétique étant de préférence choisi dans le groupe formé par les traitements antimicrobiens, antifongiques en particulier le traitement de Malassezia, des pellicules sèches et/ou grasses.

En outre, la présente invention concerne aussi à titre de médicament une composition contenant une combinaison d'au moins un rétinoïde et d'au moins un aldéhyde linéaire, le rétinoïde étant de préférence le rétinaldéhyde, l'aldéhyde linéaire comprenant de 6 à 12 atomes de carbone bornes incluses, de préférence 11 atomes de carbone en particulier l'aldéhyde linéaire à 11 atomes de carbone est l'aldéhyde undécylique, l'aldéhyde undécylénique ou un mélange de ces deux aldéhydes.

Cette composition à titre de médicament comprend de préférence une concentration en rétinaldéhyde comprise entre 0,001 et 0,5%, de préférence entre 0,015 et 0,25% en poids par rapport au poids total de la composition, et une concentration en aldéhyde linéaire comprenant 11 atomes de carbone comprise entre 0,001 et 0,5%, de préférence comprise entre 0,03 et 0,5% en poids par rapport au poids total de la composition.

Ledit médicament peut en outre comprendre au moins un solvant choisi dans le groupe formé par les aldéhydes inférieurs (c'est-à-dire comprenant de 1 à 6 atomes de carbone) tels que le méthylal, les alcools inférieurs (c'est-à-dire comprenant de 1 à 6 atomes de carbone) tel que l'éthanol, les isoparaffines et plus particulièrement les chaînes comprenant de 6 à 12 atomes de carbones bornes incluses, les silicones linéaires et les silicones cycliques, et notamment le décaméthylcyclopentasiloxane, le diméthylpolysiloxane, l'hexaméthyldisiloxane.

La présente invention vise encore un procédé de préparation du médicament selon la présente invention telle qu'on mélange au moins un rétinoïde, au moins un aldéhyde linéaire et au moins un excipient pharmaceutiquement acceptable.

Ledit médicament selon la présente invention est plus particulièrement destiné à un traitement choisi parmi les traitements antifongiques, antimicrobiens, en particulier le traitement de Malassezia, le traitement des pellicules sèches et/ou grasses.

Les auteurs de la présente invention ont mis en évidence une activité antifongique synergique lorsque l'on associe le rétinaldéhyde à certains aldéhydes linéaires et, plus particulièrement, les aldéhydes linéaires présentant une chaîne de longueur comprise entre 6 et 12 atomes de carbone tels que l'hexanal (C6), l'octanal (C8), la nonanal (C9), le décanal (C10), l'undécanal ou aldéhyde undécylique (C11), le 10-undécen-1-al (C11 UD) ou aldéhyde undécylinique, le dodécanal (C12 LAUR), le méthylxonyl acétaldéhyde (C12 MNA).

Pris isolément, ces aldéhydes présentent des activités particulièrement intéressantes sur Malassezia.

Le tableau 1 montre de manière significative l'activité de aldéhydes sur Malassezia que la souche soit référencée (IP 1363-82) ou bien sauvage.

L'invention réside dans l'observation que la combinaison du rétinaldéhyde avec ces aldéhydes, et notamment les aldéhydes en C11, permet d'aboutir à une synergie d'action antifongique très intéressante sur Malassezia furfur.

De préférence, on choisira d'une part le rétinaldéhyde et d'autre part au moins un aldéhyde linéaire C11.

Dans les compositions conformes à l'invention, la concentration en rétinaldéhyde est de préférence située dans l'intervalle compris entre 0,001 et 0,5 %, de préférence située entre 0,015 et 0,25%. Les aldéhydes linéaires en C11 seront présents à des concentrations comprises entre 0,001 et 0,5% de préférence entre 0,03 et 0,5%. Ces concentrations sont exprimées en pourcentage de poids par rapport au poids total de la composition.

Le niveau de l'efficacité a été prouvé sur des souches de Malassezia sauvages et référencées (Malassezia furfur IP163-82). De plus, les formules avec les deux actifs et avec un seul des deux actifs se distinguent de l'excipient sans activité inhibitrice intrinsèque sur les germes cités.

Les exemples qui suivent sont destinés à illustrer l'invention

Matériel et méthode :

Matériel.

| *souche : | Malassézia furfur IP 1363-82 Malassezia furfur sauvage collecté auprès d'un pityriasis versicolor chez un patient hospitalisé au Centre Hospitalier Régional de Toulouse Rangueil (France). |
|---|---|
| *milieu de culture : | Dixon modifié (sous forme de bouillon et sous forme gélosée par ajout d'agar) |
| *appareillage : | microplaques stériles Nunc ® Nunclon à 96 puits eau distillée stérile tubes à essais stériles étuve à 32°C spectrophotomètre et cuves adaptées billes de verre stériles |
| *produits : | les solutions-mères des produits cités ci-dessous ont été préparés comme suit : |

Trans rétinal :solution à 1% dans 20% d'éthanol 95 complétée avec de l'eau distillée stérile.
undécanal :solution à 1% dans 10% de Tween 80 complétée par de l'eau distillée stérile.

Méthodes : Recherche de la concentration minimale fongistatique :

- une distribution de 100µl de bouillon Dixon est réalisée dans chaque cupule de la microplaque stérile.
- une distribution de 100µl des différents produits a lieu dans une cupule, une cupule par produit (on est alors à 50% d'actif).
- une série de dilutions sériées au demi est ensuite réalisée contre un témoin stérilité du milieu de culture et un autre témoin de la vigueur de la souche.

L'ensemencement de la microplaque a lieu sous atmosphère stérile après préparation de l'inoculum fongique comme suit :
Les souches ayant préalablement repiquées 72 à 48 heures sur gélose Dixon, un inoculum de $10^7$ à $10^8$ germes par ml est dispersé dans l'eau distillée stérile, puis agité au vortex. Les billes de verre assurant une bonne dispersion des souches, la mesure de la densité optique permet de préparer un inoculum adéquat par rapport à un étalonnage préétabli et validé.
La répartition de la souche a lieu dans une deuxième microplaque stérile, par distribution de 100µl d'inoculum par microcupule.
L'ensemencement a lieu par un repiquage de la deuxième microplaque vers la première microplaque, à l'aide d'un multi ensemenceur stérile, il se réalise ainsi une dilution au 1/100 de l'inoculum : la microptaque contenant alors environ $10^5$ à $10^6$ germes par ml peut être alors mise à l'étuve à 32°C pour croissance pendant 48 à 72 heures.
La lecture de la plus grande dilution donnant lieu à une clarté identique au témoin stérilité du milieu, constitue la concentration minimale fongistatique (CMF).

Etude de la combinaison :

Des solutions-mères comme décrites précédemment égales à deux fois la CMF sont réalisées. La méthode de l'échiquier rapportée sur microplaque est utilisée pour croiser les séries de dilution au demi de chaque actif. Après ensemencement et incubation à 32°C, on procède à la lecture des CMF. Un repérage des CMF donnant lieu à des synergies peut alors se faire. Nous obtenons après calcul des FIC Index le tableau suivant :
La synergie peut être mise en évidence par le calcul des Index FIC (Fractional Inhibitory Concentration) des produits. Pour cela, on mesure la valeur de la CMF (ou Concentration Minimale Fongistatique) des principes actifs à tester vis-à-vis d'un type de germe.
Le FIC d'un produit A est défini comme :

$$FIC_A = \frac{\text{CMF du produit A en association}}{\text{CMF du produit A seul}}$$

L'association de deux antifongiques A et B est synergique si la somme FIC des rapports ($FIC_A$ et $FIC_B$) est inférieure ou égale à 0,75.
Plus la valeur FIC est faible, plus la synergie est importante.
On considère qu'il y a simple additivité pour des valeurs de FIC comprises entre 0,75 et 1,1 et indifférente dans l'intervalle compris entre 1,1 et 2 ; au-delà, l'association est notée antagoniste.

| | CMF % | | | | |
|---|---|---|---|---|---|
| Produits | Seuls | Associés | FIC | | Index |
| Rétinal | 0,25 – 0,5 | 0,06 – 0,25 | 0,51 | à | 0,75 |
| Ald. C11 | 0,06 – 0,007 | 0,0001 – 0,03 | | | |

La combinaison du rétinaldéhyde avec l'un des aldéhydes décrit précédemment sera préférentiellement vectorisée dans un milieu anhydre. Ce milieu sera plus particulièrement représenté par des solvants organiques possédant un degré de volatilité élevé. Les solvants retenus possèdent l'avantage de garantir la stabilité du rétinaldéhyde utilisé seul ou en association. Parmi les solvants reconnus intéressants, nous citerons les isoparaffines et plus particulièrement les chaînes de $C_6$ à $C_{12}$, les silicones linéaires à savoir les diméthylpolysiloxanes d'une viscosité allant de 0,65 cp à 5 cp, les structures siliconées cycliques et notamment le décaméthylcyclopentasiloxane ou encore le méthylal ou l'éthanol.

A titre indicatif, nous citerons quelques exemples de lotions topiques. Elles pourront éventuellement faire l'objet d'une distribution par le biais du système « air less » offert par l'aérosol à poche ou sous forme d'aérosol classique pressurisé par l'azote ou tout gaz liquéfié chimiquement compatible avec la formulation décrite.

**Exemple 1 : lotion**

| | | |
|---|---|---|
| rétinaldéhyde | | 0,010 à 0,10 g |
| Hexanal | | 0,01 à 1 g |
| Décaméthylcyclopentasiloxane | | 50 g |
| Isoparaffine C8-C9 | QSP | 100 g |

**Exemple 2 : lotion**

| | | |
|---|---|---|
| rétinaldéhyde | | 0,010 à 0,10 g |
| Aldéhyde C11 undécylénique | | 0,01 à 1 g |
| Butyl Hydroxy Toluène | | 0,01 g |
| Diméthylpolysiloxane | | 50 g |
| Isoparaffine C8-C9 | QSP | 100 g |

**Exemple 3 : lotion**

| | | |
|---|---|---|
| rétinaldéhyde | | 0,010 à 0,10 g |
| Butyl Hydroxy Toluène | | 0,010 g |
| Aldéhyde Undecylénique C11 | | 0,10 à 1 g |
| Citral | | 0,2 g |
| Hexaméthyldisiloxane | | 20 g |
| Décaméthylcyclopentasiloxane | | 30 g |
| Isoparaffine C8 C9 | QSP | 100 g |

**EP 1 233 748 B1**

**Exemple 4 : lotion**

| rétinaldéhyde | | 0,010 à 0,10 g |
|---|---|---|
| Hexanal | | 0,05 g |
| Aldéhyde C11 undecylénique | | 0,10 g |
| Butyl Hydroxy Toluène | | 0,015 g |
| Ethanol | | 15 g |
| Décamethylcypentasiloxane | | 45 g |
| Méthylal | QSP | 100 g |

**Revendications**

1. Composition cosmétique contenant une combinaison d'au moins un rétinoïde et d'au moins un aldéhyde linéaire associée à au moins un excipient cosmétiquement acceptable.

2. Composition cosmétique selon la revendication 1 **caractérisée en ce que** le rétinoïde est le rétinaldéhyde.

3. Composition cosmétique selon l'une des revendications 1 ou 2 **caractérisée en ce que** l'aldéhyde linéaire comprend de 6 à 12 atomes de carbone, bornes incluses.

4. Composition cosmétique selon 1a revendication 3 **caractérisée en ce que** l'aldéhyde linéaire comprend 11 atomes de carbone, de préférence l'aldéhyde linéaire est l'aldéhyde undécylique, l'aldéhyde undécylénique ou un mélange de ces deux aldéhydes.

5. Composition cosmétique selon l'une des revendications 2 à 4 **caractérisée en ce que** la concentration en rétinaldéhyde est comprise entre 0,001% et 0,5% en poids par rapport au poids total de la composition.

6. Composition cosmétique selon l'une des revendications 2 à 5 **caractérisée en ce que** la concentration en aldéhyde linéaire comprenant 11 atomes de carbone est comprise entre 0,001 % et 0,5% en poids par rapport au poids total de la composition.

7. Composition cosmétique selon l'une des revendications 4 à 6 **caractérisée en ce que** la concentration en rétinaldéhyde est comprise entre 0,015% et 0,25% et la concentration en aldéhyde linéaire est comprise entre 0,03 et 0,5% en poids par rapport au poids total de la composition.

8. Composition cosmétique selon l'une des revendications précédentes **caractérisée en ce qu'**elle contient au moins un solvant choisi dans le groupe formé par les aldéhydes inférieurs tel que le méthylal, les alcools inférieurs tel que l'éthanol, les isoparaffines et plus particulièrement les isoparaffines comprenant de 6 à 12 atomes de carbone bornes incluses, les silicones linéaires et les silicones cycliques.

9. Procédé de préparation de la composition cosmétique selon l'une des revendications précédentes **caractérisé en ce qu'**on mélange au moins un rétinoïde, au moins un aldéhyde linéaire et au moins un excipient cosmétiquement acceptable.

10. Procédé sur la peau ou le cuir chevelu de traitement cosmétique comprenant l'application topique d'une composition selon l'une des revendications 1 à 8.

11. Procédé de traitement cosmétique selon la revendication 10 **caractérisé en ce que** ledit traitement est choisi dans le groupe formé par les traitements antimicrobiens, antifongiques, en particulier le traitement de Malassezia, le traitement des pellicules sèches et/ou grasses.

12. A titre de médicament, une composition contenant une combinaison d'au moins un rétinoïde et d'au moins un aldéhyde linéaire.

**13.** A titre de médicament, une composition selon la revendication 12 **caractérisée en ce que** le rétinoïde est le rétinaldéhyde.

**14.** A titre de médicament, une composition selon l'une des revendications 12 ou 13 **caractérisée en ce que** l'aldéhyde linéaire comprend de 6 à 12 atomes de carbone, bornes incluses.

**15.** A titre de médicament, une composition selon la revendication 14 **caractérisée en ce que** l'aldéhyde linéaire comprend 11 atomes de carbone, de préférence l'aldéhyde linéaire est l'aldéhyde undécylique, l'aldéhyde undécylénique ou un mélange de ces deux aldéhydes.

**16.** A titre de médicament, une composition selon l'une des revendications 13 à 15 **caractérisée en ce que** la concentration en rétinaldéhyde est comprise entre 0,001% et 0,5% en poids par rapport au poids total de la composition.

**17.** A titre de médicament, une composition selon l'une des revendications 13 à 16 **caractérisée en ce que** la concentration en aldéhyde linéaire comprenant 11 atomes de carbone est comprise entre 0,001% et 0,5% en poids par rapport au poids total de la composition.

**18.** A titre de médicament, une composition selon l'une des revendications 13 à 17 **caractérisée en ce que** la concentration en rétinaldéhyde est comprise entre 0,015% et 0,25% et la concentration en aldéhyde linéaire est comprise entre 0,03 et 0,5% en poids par rapport au poids total de la composition.

**19.** A titre de médicament, une composition selon l'une des revendications 12 à 18 **caractérisée en ce qu'**elle contient au moins un solvant choisi dans le groupe formé par les aldéhydes inférieurs tel que le méthylal, les alcools inférieurs tel que l'éthanol, les isoparaffines et plus particulièrement les isoparaffines comprenant de 6 à 12 atomes de carbone bornes incluses, les silicones linéaires et les silicones cycliques

**20.** Composition selon l'une des revendications 12 à 19, à titre de médicament pour au -moins un traitement choisi parmi les traitements d'antifongiques, antimicrobiens, en particulier le traitement de Malassezia, le traitement des pellicules sèches et/ou grasses.

**21.** Procédé de fabrication d'un médicament selon l'une des revendications 12 à 20 **caractérisé en ce qu'**on mélange au moins un rétinoïde, au moins un aldéhyde linéaire et au moins un excipient pharmaceutiquement acceptable.

## Claims

**1.** Cosmetic composition containing a combination of at least one retinoid and of at least one linear aldehyde combined with at least one cosmetically acceptable excipient.

**2.** Cosmetic composition according to Claim 1, **characterized in that** the retinoid is retinaldehyde.

**3.** Cosmetic composition according to either of Claims 1 and 2, **characterized in that** the linear aldehyde contains from 6 to 12 carbon atoms, limits inclusive.

**4.** Cosmetic composition according to Claim 3, **characterized in that** the linear aldehyde contains 11 carbon atoms, and the linear aldehyde is preferably undecyl aldehyde, undecylenyl aldehyde or a mixture of these two aldehydes.

**5.** Cosmetic composition according to one of Claims 2 to 4, **characterized in that** the retinaldehyde concentration is between 0.001% and 0.5% by weight relative to the total weight of the composition.

**6.** Cosmetic composition according to one of Claims 2 to 5, **characterized in that** the concentration of linear aldehyde containing 11 carbon atoms is between 0.001% and 0.5% by weight relative to the total weight of the composition.

**7.** Cosmetic composition according to one of Claims 4 to 6, **characterized in that** the retinaldehyde concentration is between 0.015% and 0.25% and the concentration of linear aldehyde is between 0.03% and 0.5% by weight relative to the total weight of the composition.

8. Cosmetic composition according to one of the preceding claims, **characterized in that** it contains at least one solvent chosen from the group formed by lower aldehydes such as methylal, lower alcohols such as ethanol, isoparaffins and more particularly isoparaffins containing from 6 to 12 carbon atoms, limits inclusive, linear silicones and cyclic silicones.

9. Process for preparing the cosmetic composition according to one of the preceding claims, **characterized in that** at least one retinoid, at least one linear aldehyde and at least one cosmetically acceptable excipient are mixed together.

10. Cosmetic treatment process for the skin or the scalp, comprising the topical application of a composition according to one of Claims 1 to 8.

11. Cosmetic treatment process according to Claim 10, **characterized in that** the said treatment is chosen from the group formed by an antimicrobial treatment, an antifungal treatment, in particular treatment against Malassezia, and treatment of dry and/or greasy dandruff.

12. As a medicament, a composition containing a combination of at least one retinoid and of at least one linear aldehyde.

13. As a medicament, a composition according to Claim 12, **characterized in that** the retinoid is retinaldehyde.

14. As a medicament, a composition according to either of Claims 12 and 13, **characterized in that** the linear aldehyde contains from 6 to 12 carbon atoms, limits inclusive.

15. As a medicament, a composition according to Claim 14, **characterized in that** the linear aldehyde contains 11 carbon atoms, and the linear aldehyde is preferably undecyl aldehyde, undecylenyl aldehyde or a mixture of these two aldehydes.

16. As a medicament, a composition according to one of Claims 13 to 15, **characterized in that** the retinaldehyde concentration is between 0.001% and 0.5% by weight relative to the total weight of the composition.

17. As a medicament, a composition according to one of Claims 13 to 16, **characterized in that** the concentration of linear aldehyde containing 11 carbon atoms is between 0.001% and 0.5% by weight relative to the total weight of the composition.

18. As a medicament, a composition according to one of Claims 13 to 17, **characterized in that** the retinaldehyde concentration is between 0.015% and 0.25% and the concentration of linear aldehyde is between 0.03% and 0.5% by weight relative to the total weight of the composition.

19. As a medicament, a composition according to one of Claims 12 to 18, **characterized in that** it contains at least one solvent chosen from the group formed by lower aldehydes such as methylal, lower alcohols such as ethanol, isoparaffins and more particularly isoparaffins containing from 6 to 12 carbon atoms, limits inclusive, linear silicones and cyclic silicones.

20. Composition according to one of Claims 12 to 19, as medicinal product for at least one treatment chosen from an antifungal treatment, an antimicrobial treatment, in particular treatment against Malassezia, and treatment of dry and/or greasy dandruff.

21. Process for manufacturing a medicinal product according to one of Claims 12 to 20, **characterized in that** at least one retinoid, at least one linear aldehyde and at least one pharmaceutically acceptable excipient are mixed together.

**Patentansprüche**

1. Kosmetische Zusammensetzung, enthaltend eine Kombination von wenigstens einem Retinoid und wenigstens einem linearen Aldehyd, welche mit wenigstens einem kosmetisch annehmbaren Träger kombiniert ist.

2. Kosmetische Zusammensetzung nach Anspruch 1, **dadurch gekennzeichnet, dass** das Retinoid Retinaldehyd ist.

3. Kosmetische Zusammensetzung nach einem der Ansprüche 1 oder 2, **dadurch gekennzeichnet, dass** der lineare Aldehyd 6 bis 12 Kohlenstoffatome, Randatome eingeschlossen, umfasst.

4. Kosmetische Zusammensetzung nach Anspruch 3, **dadurch gekennzeichnet, dass** der lineare Aldehyd 11 Kohlenstoffatome umfasst, der lineare Aldehyd vorzugsweise Undecylaldehyd, Undecylenaldehyd oder eine Mischung von diesen beiden Aldehyden ist.

5. Kosmetische Zusammensetzung nach einem der Ansprüche 2 bis 4, **dadurch gekennzeichnet, dass** die Konzentration von Retinaldehyd zwischen 0,001 Gew.-% und 0,5 Gew.-% eingeschlossen bezogen auf das Gesamtgewicht der Zusammensetzung beträgt.

6. Kosmetische Zusammensetzung nach einem der Ansprüche 2 bis 5, **dadurch gekennzeichnet, dass** die Konzentration von linearem Aldehyd, welcher 11 Kohlenstoffatome umfasst, zwischen 0,001 Gew.-% und 0,5 Gew.-% eingeschlossen bezogen auf das Gesamtgewicht der Zusammensetzung beträgt.

7. Kosmetische Zusammensetzung nach einem der Ansprüche 4 bis 6, **dadurch gekennzeichnet, dass** die Konzentration von Retinaldehyd zwischen 0,015 Gew.-% und 0,25 Gew.-% eingeschlossen beträgt und die Konzentration von linearem Aldehyd zwischen 0,03 und 0,5 Gew.-% eingeschlossen bezogen auf das Gesamtgewicht der Zusammensetzung beträgt.

8. Kosmetische Zusammensetzung nach einem der vorangegangenen Ansprüche, **dadurch gekennzeichnet, dass** sie wenigstens ein Lösemittel enthält, welches ausgewählt wird aus der Gruppe, welche aus den niederen Aldehyden, wie Methylal, den niederen Alkoholen, wie Ethanol, den Isoparaffinen und insbesondere den Isoparaffinen, welche 6 bis 12 Kohlenstoffatome, Randatome eingeschlossen, umfassen, den linearen Siliconen und den cyclischen Siliconen gebildet wird.

9. Verfahren zur Herstellung der kosmetischen Zusammensetzung nach einem der vorangegangenen Ansprüche, **dadurch gekennzeichnet, dass** man wenigstens ein Retinoid, wenigstens einen linearen Aldehyd und wenigstens einen kosmetisch annehmbaren Träger mischt.

10. Verfahren zur kosmetischen Behandlung auf der Haut oder der Kopfhaut, umfassend das topische Auftragen einer Zusammensetzung nach einem der Ansprüche 1 bis 8.

11. Verfahren zur kosmetischen Behandlung nach Anspruch 10, **dadurch gekennzeichnet, dass** die Behandlung ausgewählt wird aus der Gruppe, welche aus den antimikrobiellen, antifungalen Behandlungen, insbesondere der Behandlung von Malassezia, der Behandlung von trockenen und/oder fettigen Kopfschuppen gebildet wird.

12. Zusammensetzung, welche eine Kombination von wenigstens einem Retinoid und wenigstens einem linearen Aldehyd enthält, als Arzneimittel.

13. Zusammensetzung als Arzneimittel nach Anspruch 12, **dadurch gekennzeichnet, dass** das Retinoid Retinaldehyd ist.

14. Zusammensetzung als Arzneimittel nach einem der Ansprüche 12 oder 13, **dadurch gekennzeichnet, dass** der lineare Aldehyd 6 bis 12 Kohlenstoffatome, Randatome eingeschlossen, umfasst.

15. Zusammensetzung als Arzneimittel nach Anspruch 14, **dadurch gekennzeichnet, dass** der lineare Aldehyd 11 Kohlenstoffatome umfasst, der lineare Aldehyd vorzugsweise Undecylaldehyd, Undecylenaldehyd oder eine Mischung von diesen beiden Aldehyden ist.

16. Zusammensetzung als Arzneimittel nach einem der Ansprüche 13 bis 15, **dadurch gekennzeichnet, dass** die Konzentration von Retinaldehyd zwischen 0,001 Gew.-% und 0,5 Gew.-% eingeschlossen bezogen auf das Gesamtgewicht der Zusammensetzung beträgt.

17. Zusammensetzung als Arzneimittel nach einem der Ansprüche 13 bis 16, **dadurch gekennzeichnet, dass** die Konzentration von linearem Aldehyd, welcher 11 Kohlenstoffatome umfasst, zwischen 0,001 Gew.-% und 0,5 Gew.-% eingeschlossen bezogen auf das Gesamtgewicht der Zusammensetzung beträgt.

18. Zusammensetzung als Arzneimittel nach einem der Ansprüche 13 bis 17, **dadurch gekennzeichnet, dass** die Konzentration von Retinaldehyd zwischen 0,015 Gew.-% und 0,25 Gew.-% eingeschlossen bezogen auf das Gesamtgewicht der Zusammensetzung beträgt und die Konzentration von linearem Aldehyd zwischen 0,03 und 0,5 Gew.-% eingeschlossen bezogen auf das Gesamtgewicht der Zusammensetzung beträgt.

19. Zusammensetzung als Arzneimittel nach einem der Ansprüche 12 bis 18, **dadurch gekennzeichnet, dass** sie wenigstens ein Lösemittel enthält, welches ausgewählt wird aus der Gruppe, welche aus den niederen Aldehyden, wie Methylal, den niederen Alkoholen, wie Ethanol, den Isoparaffinen und insbesondere den Isoparaffinen, welche 6 bis 12 Kohlenstoffatome, Randatome eingeschlossen, umfassen, den linearen Siliconen und den cyclischen Siliconen gebildet wird.

20. Zusammensetzung nach einem der Ansprüche 12 bis 19 als Arzneimittel für wenigstens eine Behandlung, welche aus den antifungalen, antimikrobiellen Behandlungen, insbesondere der Behandlung von Malassezia, der Behandlung von trockenen und/oder fettigen Kopfschuppen gebildet wird.

21. Verfahren zur Herstellung eine Arzneimittels nach einem der Ansprüche 12 bis 20, **dadurch gekennzeichnet, dass** man wenigstens ein Retinoid, wenigstens einen linearen Aldehyd und wenigstens einen pharmazeutisch annehmbaren Träger mischt.